# EUROPEAN PATENT SPECIFICATION

(11) **EP 2 806 807 B1**
(45) Date of publication and mention of the grant of the patent: **09.08.2017**
(21) Application number: 13704374.1
(22) Date of filing: 23.01.2013
(51) Int. Cl.: A61B 17/16, A61B 17/88, A61B 17/80

(54) **SURGICAL TOOL SYSTEM**
CHIRURGISCHES WERKZEUGSYSTEM
SYSTÈME D'OUTIL CHIRURGICAL

(30) Priority: 23.01.2012 EP 12152057
(43) Date of publication of application: 03.12.2014
(73) Proprietor: Universität Zürich, 8006 Zürich (CH)
(72) Inventor: CIRITSIS, Bernhard, Dimitris, 8006 Zürich (CH)
(74) Representative: Schulz Junghans Patentanwälte PartGmbB
(86) International application number: PCT/EP2013/051206
(87) International publication number: WO 2013/110638

(56) References cited:
- US-A1- 2004 106 997

## Description

The invention relates to a surgical tool for performing surgery on a bone, particularly for reinforcing the bone in a miniinvasive manner.

Osteoporotic fractures, especially in the femoral neck area are already a challenging surgical problem that will become even more important in the next future because of the growing percentage of elderly people in the western societies.

Already in 1990 there were more than 1,7 Million cases of femoral neck fractures worldwide. The life time incident rate is between 10% and 20%.

A study recently published by the WHO shows that the fracture of the femoral neck is one of the ten most severe pathologies worldwide, causing death in up to 25% of the patients in one year after the fracture. More than 50% of the patients will be chronically invalid after a fracture of the femoral neck and 25% need continuously nursing.

The risk of fracture of the controlateral side after a first femoral neck fracture is 6 to 9 times higher than the life time incident rate.

The socioeconomic impact for this pathology is enormous and still growing: In 2000, alone in Switzerland, the costs for acute hospital care were estimated to amount to more than 95 Million Swiss Francs.

Up to now, the prior art to resolve the pathology of femoral neck fractures is to realize an osteosynthesis or to replace the femoral head by a prosthesis. Both ways are, as mentioned above, linked to high costs and high morbidity and mortality.

Further, US 2004/106997A1 describes a method and apparatus for creating safer and less intrusive surgical channels or pathways. The dilatory apparatus includes a guide wire, two or more cannulated dilators, and a guide tube.

Therefore, the problem underlying the present invention is to provide for a surgical tool that helps in preventing fracture due to osteoporosis, particularly in a miniinvasive way, meaning very low blood loss and a short time of surgical intervention.

This problem is solved by a surgical tool having the features of claim 1.

According thereto, the surgical tool comprises: a first and a second element each extending along a longitudinal axis, wherein the two elements each comprise a shell encompassing a hollow space of the respective element, wherein the second element is designed to be inserted into the hollow space of the first element in order to arrange the two elements in an embedded state, in which the two elements are arranged coaxially with respect to each other and in which the shell of the first element encompasses the shell of the second element, wherein particularly the second element being arranged in said embedded state is designed to be pushed with a first free end of the second element ahead through pre-incised tissue towards a bone to be reinforced so as to make cortical contact with said bone; a guiding element (e.g. guiding wire) extending along a longitudinal axis, wherein the guiding element comprises a first free end, and wherein the guiding element is designed to be inserted into the hollow space of the second element with said first free end ahead in order to arrange the guiding element in an inserted state with respect to the second element when the latter is arranged in said embedded state, in which inserted state the guiding element is arranged coaxially with respect to the second element and encompassed by the shell of the second element, and wherein the guiding element is designed to be drilled into a bone to be treated with its first free end ahead when being arranged in said inserted state so as to fasten said guiding element to said bone; and a drill extending along a longitudinal axis, which drill comprises a shell encompassing a hollow space of the drill, wherein the drill, particularly after having removed the second element from the first element and from the guiding element, is designed to be put over the guiding element with a drill head ahead in order to arrange the drill in a drilling state with respect to the guiding element and the first element, in which the first element encompasses said drill for tissue protection and in which the guiding element is arranged in the hollow space of the drill so as to guide said drill upon drilling, and wherein the drill head is designed to drill a borehole into the corticalis and the spongiosa of said bone in said drilling state.

Since the afore-described surgical tool comprises a number of separate components interacting with each other one may also speak of a surgical tool system.

Advantageously, the tool according to the invention particularly allows for getting access to the spongiosa of a bone, particularly the femur, in a defined and precise manner, thus laying the foundation for reinforcing the bone in order to prevent it from fracture.

Further, the tool according to the invention can be used to realize (X-ray assisted) biopsy out of an e.g. tumerous bone or to get access to another kind of osteolytic zone and to reinforce it, even to place medicaments into a bone.

The tool according to the invention is particularly suited for the femoral neck but can also be used for other (big) bones in the human body.

Preferably, the first and second element, the guiding element, as well as the drill (and other elements described below) are made of (e.g. surgical) steel.

Further, the shells of the first and the second element as well as the shell of the drill are particularly formed (at least in sections) hollow circular cylindrical. Preferably, the guiding element is formed at least in sections as a circular cylinder. Thus, said longitudinal axes of the afore-described elements are preferably cylinder axes. Correspondingly, the hollow spaces are shaped (at least in sections) cylindrical. Further, at the first free end of the guiding element, a drill head is particularly provided for drilling the guiding element into the bone, wherein particularly said drill head may be tapered towards the first free end of the guiding element.

Preferably, the second element, particularly after removal of the drill and the first element from the guiding element and re-arrangement of the second element over the guiding element such that the latter is arranged in the hollow space of the second element, is designed to be inserted with its free end into said borehole (i.e. into a corticalis section of said borehole drilled by means of said drill), particularly so as to bridge the corticalis of said bone, i.e., the spongiosa lying beneath the corticalis of said bone is then (directly) accessible via the hollow space of the second element.

Further, the tool according to the invention further comprises a cannula (also made of (surgical) steel, preferably), which cannula extends along a longitudinal axis, wherein the cannula comprises a shell encompassing a hollow space of the cannula, through which bone cement can be piped that is to be injected into said borehole, wherein the cannula is designed to be inserted into the hollow space of the second element (for serving as a guiding means for the cannula) with a first free end of the cannula ahead in order to arrange the cannula in an injection state with respect to the second element, particularly after the guiding element has been removed from the second element, in which injection state the cannula is arranged coaxially with respect to the second element and encompassed by the shell of the second element.

Preferably also the cannula is formed as a hollow circular cylinder such that its longitudinal axis is a cylinder axis. Correspondingly the hollow space of the cannula is formed cylindrical in particular. The cannula may be single use.

Preferably, the second element can be inserted into the first element in a form fitting manner. Particularly, a slight play can be present between the two engaged elements that is needed for being able to easily slide the second element back and forth with respect to the first element manually. Likewise, the cannula is preferably insertable into the hollow space of the second element in an essentially form-fitting manner, the guiding element is preferably insertable into the hollow space of the second element or of the drill in an essentially form-fitting manner, the drill is preferably insertable in the hollow space of the first element in an essentially form-fitting manner. A typical play may have a range between 0.0mm (no play at all) and 0.5mm.

Further, the cannula is preferably designed to be inserted into the second element such that it projects with its first free end past the hollow space of the second element (i.e. out of an opening delimited by the first free end of the second element), particularly through said borehole into the spongiosa of the bone, wherein particularly said first free end of the cannula delimits an injection opening of the cannula, through which bone cement can be discharged out of the hollow space of the cannula into the borehole, i.e., through the corticalis into the spongiosa in order to generate a bone cement implant reinforcing the bone. The cannula is further designed to be movable inside the hollow space of the second element, so that it can be retracted upon injecting bone cement into the spongiosa.

Particularly, the tool further comprises a stamper, particularly made from (surgical) steel, extending along a longitudinal axis, wherein the stamper is designed to be inserted into the hollow space of the cannula with a first free end of the stamper ahead, particularly in a form-fitting manner, in order to push the remaining bone cement by means of said first free end out of the hollow space of the cannula, particularly through said borehole into the spongiosa of the bone. The stamper may be single use.

Particularly, the stamper being inserted in the hollow space of the cannula and the cannula are designed to be pushed together along their coaxial longitudinal axes in the hollow space of the (stationary) second element, wherein said second element is designed to guide the cannula and the stamper upon said movement, particularly so as to push bone cement injected into said borehole into the spongiosa, thereby forming a circular hollow cylindrical bone cement implant in the spongiosa of said bone.

It is to be noted that this creates a lightweight implant having a higher resistance with respect to a bending load compared to a full cylinder due to the essentially hollow cylindrical form of the implant created by said perforation of the injected bone cement.

The tool system can be adapted such concerning its dimensions that the hollow cylindrical bone cement implant comprises the desired dimensions concerning for instance the inner diameter of its hollow space or its wall thickness.

Preferably, the stamper comprises a second free end opposing the first free end along the longitudinal axis of the stamper, wherein the stamper comprises a circular plate (head) at the second free end extending perpendicular to the longitudinal axis of the stamper, from which broadened head a circular cylindrical shaft of the stamper protrudes forming the portion of the stamper that can be inserted into the hollow space of the cannula. Thus, the longitudinal axis of the stamper is also a cylinder axis in particular.

Further, when the second element is (initially) in its embedded state with respect to the first element, the first free end of the second element particularly protrudes out of the first element along its longitudinal axis (i.e. out of an opening of the first element delimited by the first free end of the first element) for making cortical contact, wherein said first free end of the second element is preferably rounded. Preferably, also the first free end of the first element is rounded to reduce the risk of injury.

Particularly, the second element comprises a second free end opposing the first free end of the second element along the longitudinal axis of the second element, wherein particularly said second free end circulates (i.e. extends circumferentially around) an opening of the second element through which said guiding element and said cannula can be inserted into the hollow space of the second element.

Preferably, the second element comprises a (free end) portion near the second free end that broadens towards said second free end, particularly such that said portion comprises at least in sections the shape of a truncated cone, wherein the second free end of the second element preferably forms a circulating (e.g. ring-like or circumferentially extending) protrusion protruding from an outside of said portion. Particularly, also the first element comprises a second free end opposing the first free end of the first element along its longitudinal axis, wherein particularly said second free end circulates (i.e. extends circumferentially around) an opening of the first element, through which the second element and said drill can be inserted into the hollow space of the first element.

For receiving the tapered portion of the second element, the hollow space of the first element preferably comprises a section along which the hollow space broadens towards said opening (second free end) of the first element, particularly such that said section of the hollow space also comprises the shape of a truncated cone.

Also here, the second free end of the first element particularly forms a circulating (e.g. ring-like or circumferentially extending) protrusion protruding from an outside of the shell of the first element.

Since the drill is a hollow member, its drill head encompasses an end portion of the hollow space of the drill. Preferably, for cutting the bone and banking of the borings upon drilling, the drill head is particularly formed as a spiral drill.

In order to be able to determine the current depth of a borehole drilled with the drill upon drilling as well as to support banking of the borings, the shell of the drill preferably comprises an outside, on which a plurality of recesses are formed extending at least in section along a circumferential direction of the drill (i.e. across the longitudinal axis of the drill), which recesses (grooves) are equidistantly distributed along the longitudinal axis of the drill.

Further, in order to be able to couple a second free end of the drill opposing the drill head of the drill along the longitudinal axis of the drill to an actuator (e.g. drilling machine) for rotating the drill about its longitudinal axis, the drill preferably comprises a hexagonal cross sectional contour at the second free end so that it may be releasably fixed in a boring socket of said actuator.

Preferably, the tool further comprises a closure element being designed to be introduced into said borehole, particularly through the cannula by means of the stamper, particularly so as to retain bone cement that was injected into the borehole (e.g. to prevent the bone cement from dripping out of the borehole).

Particularly, the closure element is designed to be introduced into said borehole in a folded state, wherein the closure element is further designed to unfold at least partially when positioned in said borehole, particularly such that the closure element butts against a surrounding wall of said borehole. Preferably, an outer diameter of the closure element then corresponds to the outer diameter of the cannula or inner diameter of the borehole.

Further, the closure element preferably comprises an outer contour which comprises a serrated shape in said folded state and preferably an octagonal shape in a completely unfolded (e.g. flat) state.

Preferably, the closure element is made out of a material or comprises a material, particularly paper, particularly sterilized Lackmus paper, allowing for detecting the closure element by means of electromagnetic radiation, particularly X-rays, wherein particularly said paper or material is dyed, particularly in standardized contrast liquid solution, so that is visible under X-ray control.

Further, the problem according to the invention is solved by a method for treating, examining and or reinforcing a bone, which may comprise the following successive steps and may use the surgical tool according to the invention.

Particularly, a patient with diagnosed osteoporosis and/or high risk of fracture of the femoral neck or another (big) bone is installed on an extension table without traction in a supine position.

Particularly, the position of the femoral neck (or another region of a bone) and the point of skin incision is controlled and validated, particularly by means of x-ray, particularly in an antero-posterior (a.p.) and/or in a lateral view.

Particularly, a small skin incision is realized over the chosen point.

Particularly, soft tissues are split by introducing a second element (also denoted as canulated candle), which again is embedded in a first element (also denoted as jacket).

Particularly, the second element together with the first element is pushed forward until there is cortical contact with the bone to be examined, treated and/or reinforced.

Particularly, the right position of said elements is controlled, particularly under x-ray.

Particularly, a guiding element (e.g. guiding wire) is drilled into the bone (e.g. femoral neck) through the second element (canulated candle).

Particularly, the right position of the guiding element is controlled, particularly under x-ray.

Particularly, the second element is extracted from the first element.

Particularly, a drill (being canulated, i.e., comprising a hollow space) is introduced over the guiding element, so as to drill a hole into the corticalis and the spongiosa of the bone (e.g. femoral neck), particularly under x-ray control. Particularly, the first element is kept in place to protect soft tissues while drilling.

Particularly, the drill is taken out of the bone, the guiding element is kept in place, and the first element is removed.

Particularly, the second element is reintroduced over the guiding element and softly tapped into the drilled hole of the corticalis of the bone (e.g. femoral neck area) featured by the drill until the corticalis is bridged by the second element.

Particularly, the position of the second element is controlled, particularly by means of x-ray, and the guiding element is taken out of the bone (e.g. femoral neck).

Particularly, a cannula is introduced through the second element, and bone cement is injected, particularly under x-ray control, into the drilled hole in the bone (e.g. femoral neck area) through said cannula.

Particularly, while injecting the bone cement, the cannula is slowly retracted, particularly under x-ray control, from the drilled hole until reaching the edge (first free end) of the second element, still fixed in the corticalis of the bone (e.g. femoral neck).

Particularly, the cannula is kept in place and a stamper, particularly of equal length, is introduced into the cannula to push the remaining bone cement in the cannula forward into the bone.

Particularly, the bone cement is perforated, particularly under x-ray control, by tapping the stamper together with the cannula gently into the bone cement implant positioned in the bone (e.g. femoral neck area) so that the bone cement is pushed into the spongiosa.

Particularly, the stamper together with the cannula is removed from the bone cement when it begins to dry while the second element is kept in place.

Particularly, the second element is removed from the corticalis of the bone (e.g. femoral neck) when the bone cement is completely dry. Particularly, the surgical field is washed out and the skin incision is closed. Particularly, a last (e.g. x-ray) control, particularly in a.p. and lateral view, is performed.

Particularly, before injecting the bone cement into the borehole a closure element is introduced into said borehole, particularly so as to retain bone cement that is to injected into the borehole. This is preferably done in case the drilled hole done by the guiding element breaks through the bone in order to prevent bone cement from dropping out of the borehole.

Particularly, the closure element is introduced into said borehole in a folded state, wherein the closure element unfolds at least partially when positioned in said borehole, particularly such that the closure element presses against a surrounding wall of said borehole or hole drilled by means of the guiding element.

Further, the closure element is preferably pushed into the borehole through the cannula with help of the stamper, particularly under visualization of the closure element by means of electromagnetic radiation, particularly X-rays, before introducing bone cement into the borehole.

Therefore, particularly, the closure element is designed to be detected by means of X-rays.

Further features and advantages of the invention shall be described by means of a detailed description of an embodiment with reference to the Figures, wherein
- Fig. 1: shows a cross sectional view of a second element (canulated candle) of a tool according to the invention,
- Fig. 2: shows a cross sectional view of a first element (metal jacket) of the tool according to the invention,
- Fig. 3: shows a cross sectional view of the first element shown in Fig. 2 in a plane perpendicular to the longitudinal axis of the first element,
- Fig. 4: shows a cross sectional view of a guiding element of the tool according to the invention,
- Fig. 5: shows a cross sectional view of a drill of the tool according to the invention,
- Fig. 6: shows a cross sectional view of the drill shown in Fig. 5 in a plane perpendicular to the longitudinal axis of the drill,
- Fig. 7: shows a cross sectional view of a cannula of the tool according to the invention
- Fig. 8: shows a cross sectional view of a stamper of the tool according to the invention,
- Fig. 9: shows a cross sectional view of the drill inserted into the first element,
- Fig. 10: shows a cross sectional view of the second element being inserted into the first element, and
- Fig. 11: shows a plan view of a closure element for closing a drilled hole.

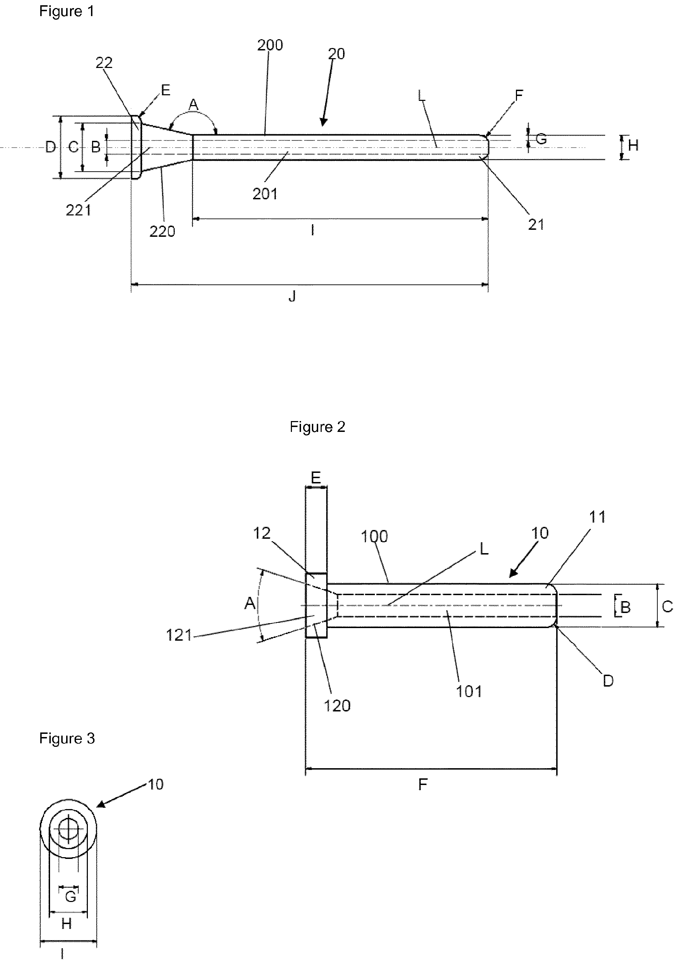

- Figs. 1 to 10: show the separate components of a tool according to the invention.

Said tool comprises an elongated first element (metal jacket) 10 as shown in Figs. 2 and 3 that comprises a shell 100 surrounding an elongated hollow space 101. The shell 100 and the hollow space 101 each extend along a longitudinal (cylinder) axis L of the first element 10.

The first element 10 comprises a first free end 11 that is rounded (curvature radius D), as well as an opposing second free end 12 that forms a circular protrusion of the first element 10. Further, for simplifying insertion of a second element 20 into the hollow space 101 of the first element 10, the hollow space 101 of the first element 10 comprises a section 120 encompassed by said protrusion 12 that broadens towards an opening 121 delimited by said protrusion (second free end) 12 in the form of a truncated cone.

The first element 10 serves for protecting tissue covering a bone that is to be reinforced by means of the tool according to the invention.

The dimensions of the first element 10 may be chosen according to Figs. 2 and 3 to be A=35°, B=10.50mm, C=20.00mm, curvature D=5.00mm, E=10.00mm, F=120.00mm, G=10.50mm, H=20.00mm, and I=30.00mm, in a second embodiment of the surgical tool F may be different, e.g. F=100.00mm, while the other dimensions A, B, C, D, E, G, H, and I of the first element 10 are preferably not changed.

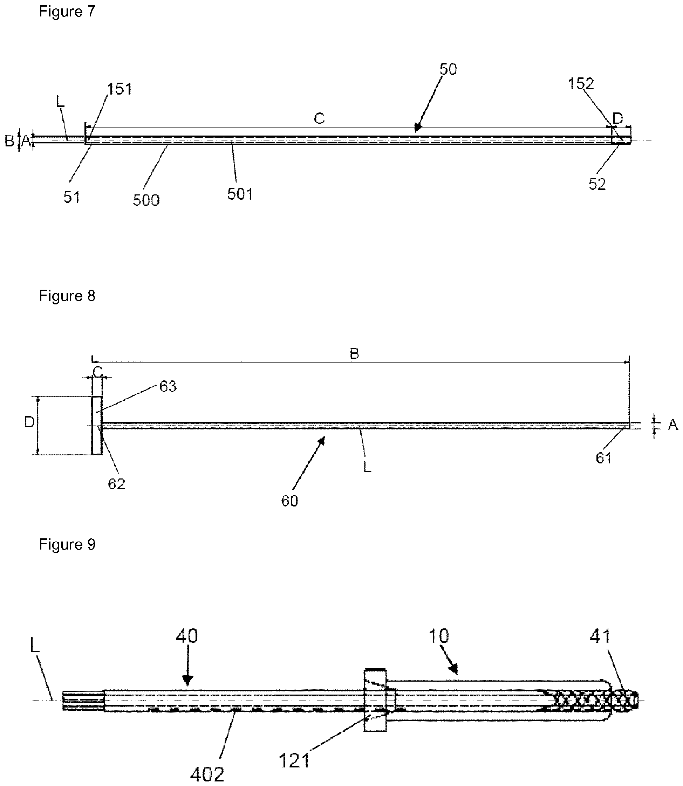

The tool further comprises a second element 20 shown in Fig. 1 that is to be housed by the first element 10 in an embedded state as shown in Fig. 9. The second element 20 also extends along a longitudinal (cylinder) axis L and comprises a shell 200 surrounding an elongated hollow space 201, too.

The second element (canulated candle) 20 is designed to be introduced into the hollow space 101 of the first element 10 with a first free end 21 of the second element 20 ahead through said opening 121 of the first element 10, wherein, when the second element 20 is fully inserted into the hollow space 101 of the first element 10, said first free end 21 of the second element 20 protrudes out of the hollow space 101 of the first element 10 through the first free end 11 of the first element 10 along its longitudinal axis L, thus providing a region of the second element 20 that serves for making cortical contact to the bone.

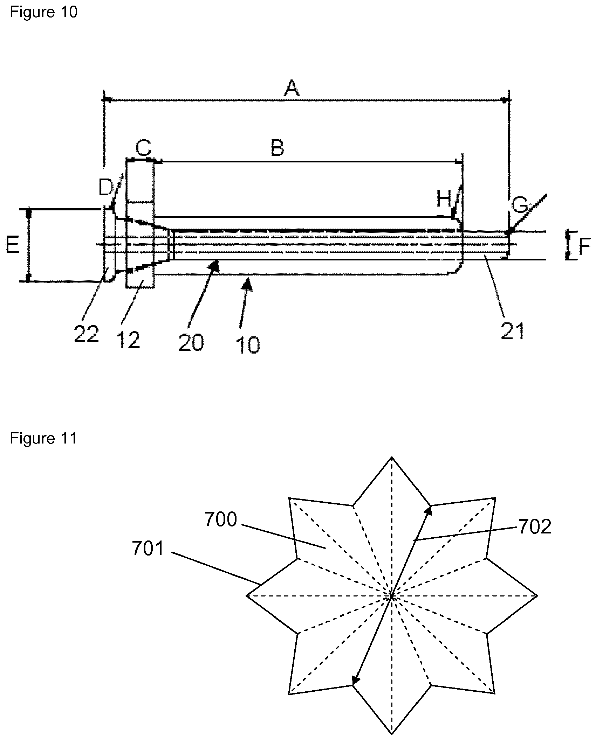

The second element 20 further comprises a second free end 22 opposing its first free end 21 along the longitudinal axis L of the second element 20 forming a circulating (e.g. circumferentially extending) protrusion 22 of the second element 20, which delimits an opening 221 of the second element 20 through which the hollow space 201 of the second element 20 is accessible. The shell 200 of the second element 20 further comprises a portion 220 that broadens towards said protrusion 22 in the form of a truncated cone, wherein said portion 220 is adapted to be received by the afore-described section 120 of the hollow space 101 of the first element, which forms a stop for the second element 20 (c.f. Fig. 10).

The dimensions of the second element 20 may be chosen according to Fig 1 to be A=167°, B=5.50mm, C=19.50mm, D=25.50mm, curvature E=2.00mm, curvature F=4.50mm, G=2.25mm, H=10.00mm, 1=120.00mm, and J=145.00mm. In the second embodiment I and J may be different, e.g. I=100.00mm and J=125.00mm, while the other dimensions A, B. C, D, E, F, G, and H of the second element 20 are preferably not changed

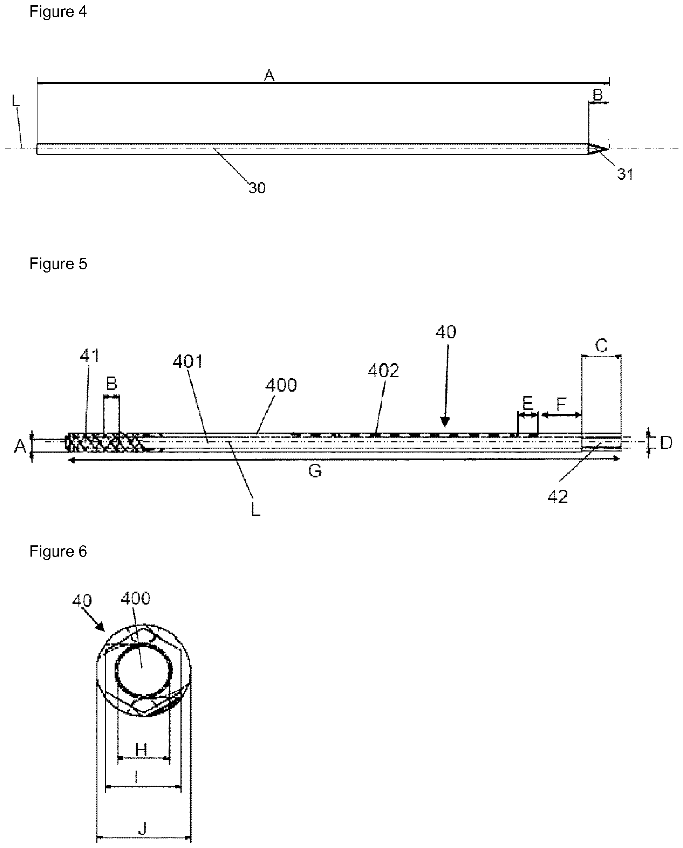

With the two elements 10, 20 being arranged in the afore-described embedded state, an elongated guiding element 30 of the tool according to the invention as shown in Fig. 4 is provided, which guiding element 30 is essentially cylindrical, extends along a longitudinal (cylinder) axis L, and is designed to be inserted with a tapering drill head 31 ahead into the hollow space 201 of the second element 20 via the opening 221 of the latter, which hollow space 201 guides the guiding element 30 towards the bone. Then, the guiding element 30 is drilled into the bone in order to anchor it thereabouts.

The dimensions of the guiding element 30 according to Fig. 4 may be chosen to be A=280,00mm, and B=10,00mm. The drill head may comprise an outer tapered Whitworth thread (e.g. of specification R1/8 - 28 according to Din 2999).

After having anchored the guiding element 30, the second element 20 is removed from the first element 10, and the guiding element 30 now provides guidance for a further component of the tool according to the invention in the form of an elongated drill 40 as shown in Figs. 5 and 6. The drill 40 comprises an essentially cylindrical shell 400 surrounding a hollow space 401 of the drill 40 extending along a longitudinal (cylinder) axis L. The drill 40 further comprises a drill head 41 at a first free end of the drill encompassing a free end portion of the hollow space 401 of the drill 40 as well as a second free end 42 opposing the drill head 41 along said longitudinal axis L, which second free end comprises a hexagonal shape in a cross section perpendicular to the longitudinal axis for clamping the drill 40 in a corresponding socket of an actuator (drilling machine, not shown).

For drilling a borehole into the corticalis and the spongiosa of the bone (e.g. femural neck), the drill 40 is inserted through the opening 121 of the first element 10, so as to arrange the drill 40 in the hollow space 101 of the first element 10, whereby the guiding element 30 is arranged in the hollow space 401 of the drill 40 to guide the latter upon drilling. At the same time, the first element 10 provides protection for the surrounding tissue. The drill 40 comprises a length along the longitudinal axis L such that the drill 40 protrudes out of the first element 10 with its drill head 41 at the first free end 11 of the first element 10 as shown in Fig. 9 (here, guiding element is not shown).

In order to control the depth of a borehole drilled by means of the drill 40 along the longitudinal axis L, the drill 40 further comprises equidistant recesses 402 on its shell 400 that also support banking of the borings.

The dimensions of the drill 40 may be chosen according to Figs. 5 and 6 to be A=6.39mm, B=7.64mm, C=20.00mm, D=5.50mm, E=11.00mm, F=22.00mm, G=280.00mm, H=5.50mm, I=9.00mm, and J=10.00mm. In the second embodiment I may be different, e.g. I=8.00 mm, while dimensions A, B, C, D, E, F, G, H, and J of drill 40 are preferably not changed. Please note, that the dimensions the drill 40 may be adapted to a specific drill chuck of a drilling machine that is employed for drilling the respective borehole.

After having drilled a borehole into the corticalis and spongiosa of the bone, the drill 40 is taken out of the bone, whereas the guiding element 30 is kept in place. The first element 10 is also removed.

Now, the second element 20 is reintroduced over the guiding element 30 and pressed into the drilled borehole of the corticalis of the bone (femoral neck area) provided by the drill 40 in beforehand until the corticalis is bridged by the (first free end of the) second element 20.

Once the second element 20 is in place (i.e., properly inserted in the borehole), the guiding element 30 is taken out of the bone (femoral neck) and an elongated cannula 50 as shown in Fig. 7 comprising a shell 500 surrounding a hollow space 501 of the cannula 50 extending along a longitudinal axis L is introduced with a first free end 51 ahead in the hollow space 201 of the second element 20, which guides the cannula 50 towards the spongiosa into which bone cement is injected through the hollow space 501 of the cannula 50 via an injection opening 151 at the first free end 51 of the cannula 50. While injecting the bone cement, the cannula 50 is retracted until it reaches the first free end 11 of the second element 20 being anchored in the corticalis part of the borehole (in the femoral neck). Further, the cannula 50 comprises a second free end 52 having an outer thread (e.g. of specification M5x0.8 - 6g), which second free end 52 delimits an opening 152 of the cannula 50, through which the hollow space 501 of the cannula 50 is accessible.

According to Fig. 7, the cannula 50 may comprise the dimensions A=3.00mm, B=5.00mm, C=270.00mm, and D=10.00mm. In the second embodiment C may be different, e.g. C=220.00mm, while the other dimensions A, B,and D of the cannula 50 are preferably not changed. Reducing the length of the cannula 50 allows for reducing the pressure of the bone cement in the cannula 50 while injecting it.

The cannula 50 is kept in place and an elongated stamper 60 according to Fig. 8 is inserted form-fittedly through said opening 152 of the cannula 50 into the hollow space 501 of the cannula 50 with a first free end 61 of the stamper 60 ahead. The stamper 60 further comprises an elongated cylindrical shaft extending along a longitudinal (cylinder) axis L, as well as a broadened head in the form of a circular plate 63 at a second free end 62 of the stamper 60 opposing the first free end 61 along the longitudinal axis L.

With the first free end 61 being inserted in the hollow space 501 of the cannula 50, remaining bone cement residing in said cannula 50 can be easily forwarded into the bone.

According to Fig. 8, the stamper 60 may comprise the dimensions A=3.00mm, B=280.00mm, C=5.00mm, and D=30.00mm. In the second embodiment, B may be different, e.g. B=230.00mm, while dimensions A, C, and D of stamper 60 are preferably not changed.

Finally, the bone cement injected into the spongiosa is pushed laterally into the spongiosa by pushing the stamper 60 together with the cannula 50 into the injected bone cement implant positioned in the bone (e.g. femoral neck area) so that the bone cement implant assumes a hollow cylindrical shape particularly providing resistance against bending loads.

The stamper 60 together with the cannula 50 are removed from the bone cement when it begins to dry, the second element 20 is kept in place.

Once the bone cement is completely dry, also the second element 20 is removed from the corticalis of the bone (femoral neck).

In case, for instance, the drilled hole done by the guiding element 30 touches the articulation surface of the femoro-acetubular joint so that the borehole becomes a through-hole through the bone (e.g. femur), a closure element 700 according Fig. 11, particularly single use, is placed into the borehole (e.g. of the femoral neck), before the introduction of the bone cement to prevent bone cement from dropping out of the borehole on the other side (e.g. into the femoro-acetubular articulation). Preferably, the closure element 700 has a serrated outer contour 701 in a folded state shown in Fig. 11 (creases are shown in dashed lines in Fig. 11), wherein the outer contour 701 preferably is an octagon when the closure element 700 is arranged in a flat unfolded state. Preferably, the closure element 700 is designed to unfold, particularly like an umbrella, at least partially after being introduced into the borehole, such that the closure element 700 presses against the surrounding wall of the borehole and thus prevents bone cement from dropping out of the borehole. The outer diameter 702 of the closure element 700 preferably corresponds to the outer diameter of the cannula 50 or inner diameter of the borehole.

Further, the closure element 700 is preferably designed to be pushed into the borehole (e.g. of the femoral neck) through the cannula 50 with help of the stamper 60, particularly under X-ray control, before introducing bone cement into the borehole. The diameter of the closure element 700 particularly corresponds to the diameter of the cannula 50.

The closure element 700 can be made out of sterilized Lackmus paper, dyed in standardized Contrast Liquid Solution to make it visible under X-ray control.

Due to Osteoporosis and the individual shape of the femoral neck spongiosa it might happen that bone cement is pushed towards the distal femoral neck area during the femoroplastic procedure, thus provoking a pistol grip shaped bone cement implant.

We suggest that this form gives hypothetically even a higher strength to the femoral neck area and prevents it of pertrochanteric fractures as a side effect of the femoroplastic procedure. The dimensions of Fig. 10 may be chosen to be A=145.00mm, B=110.00mm, C=10.00mm, curvature D=2.00mm, E=25.50mm, F=10.00mm, curvature G=4.00mm.

It is to be noted that the dimensions of the surgery tool stated above can be adapted to the individual surgical purpose if necessary.

## Claims

1. Surgical tool for performing surgery on a bone, particularly for reinforcing the bone in a miniinvasive manner, comprising:
- a first and a second element (10, 20) extending along a longitudinal axis (L), respectively, wherein the two elements (10, 20) each comprise a shell (100, 200) defining a hollow space (101, 201) of the respective element (10, 20), wherein the second element (20) is designed to be inserted into the hollow space (101) of the first element (10) along the first element's longitudinal axis (L) in order to arrange the two elements (10, 20) in an embedded state, in which the two elements (10, 20) are arranged coaxially with respect to each other and in which the shell (100) of the first element (10) encompasses the shell (200) of the second element (20), wherein particularly the second element (20) being arranged in said embedded state is designed to be pushed with a first free end (21) of the second element (20) ahead towards the bone so as to make contact with said bone,
- a guiding element (30) extending along a longitudinal axis (L), wherein the guiding element (30) comprises a first free end (31), and wherein the guiding element (30) is designed to be inserted into the hollow space (201) of the second element (20) along the second element's longitudinal axis (L) with said first free end (31) of the guiding element (30) ahead, in order to arrange the guiding element (30) in an inserted state with respect to the second element (20) when the latter is arranged in said embedded state, in which inserted state the guiding element (30) is arranged coaxially with respect to the second element (20) and encompassed by the shell (200) of the second element (20), and wherein the guiding element (30) is designed to be drilled into said bone with its first free end (31) ahead when being arranged in said inserted state so as to anchor said guiding element (31) in said bone, and
- a drill (40) extending along a longitudinal axis (L), which drill (40) comprises a shell (400) defining a hollow space (401) of the drill (40), wherein the drill (40), particularly after having removed the second element (20) from the first element (10) and from the guiding element (30), is designed to be put over the guiding element (30) along the drill's longitudinal axis (L) with a drill head (41) of the drill (40) ahead in order to arrange the drill (40) in a drilling state with respect to the guiding element (30) and the first element (10), in which drilling state the first element (10) encompasses said drill (40) for tissue protection and the guiding element (30) is arranged in the hollow space (401) of the drill (40) so as to guide said drill (40), and wherein the drill head (41) is designed to drill a borehole into the corticalis and the spongiosa of said bone in said drilling state,
**characterized in that**
the tool further comprises a cannula (50), which cannula (50) extends along a longitudinal axis (L), wherein the cannula (50) comprises a shell (500) defining a hollow space (501) of the cannula (50), through which bone cement can be piped that is to be injected into said borehole, wherein the cannula (50) is designed to be inserted into the hollow space (201) of the second element (20) along the second element's longitudinal axis (L) with a first free end (51) of the cannula (50) ahead in order to arrange the cannula (50) in an injection state with respect to the second element (20), particularly after the guiding element (30) has been removed from the second element (20), in which injection state the cannula (50) is arranged coaxially with respect to the second element (20) and encompassed by the shell (200) of the second element (20).

2. Surgical tool according to claim 1, **characterized in that** the second element (20), particularly after removal of the drill (40) and the first element (10) from the guiding element (30) and re-arrangement of the second element (20) over the guiding element (30) such that the latter is arranged in the hollow space (201) of the second element (20) again, is designed to be inserted with its first free end (21) ahead into said borehole along the second element's longitudinal axis (L), particularly so as to bridge the corticalis of said bone.

3. Surgical tool according to claim 1 or 2, **characterized in that** the cannula (50) is designed to protrude with its first free end (51) out of the hollow space (201) of the second element (20) along the second element's longitudinal axis (L), particularly into the spongiosa of the bone, wherein particularly said first free end (51) of the cannula (50) delimits an injection opening (151) of the cannula (50), through which bone cement can be discharged out of the hollow space (501) of the cannula (50).

4. Surgical tool according to claim 1 or 3, **characterized in that** the tool further comprises a stamper (60) extending along a longitudinal axis (L), wherein the stamper (60) is designed to be inserted into the hollow space (501) of the cannula (50) along the cannula's longitudinal axis (L) with a first free end (61) of the stamper (60) ahead, in order to push bone cement residing in the hollow space (501) of the cannula (50) out of said hollow space (501), particularly through said borehole into the spongiosa of the bone.

5. Surgical tool according to claim 4, **characterized in that** the stamper (60) and the cannula (50) are designed to be moved together in the hollow space (201) of the second element (20) along the second element's longitudinal axis (L) from a retracted first position into an advanced second position, particularly so as to push bone cement injected into said borehole into the spongiosa, thereby forming a hollow circular cylindrical bone cement implant in the spongiosa of said bone, wherein particularly said second element (20) is designed to guide the cannula (50) and the stamper (60) upon said movement from the retracted position into the advanced position.

6. Surgical tool according to claim 4 or 5, **characterized in that** the stamper (60) comprises a second free end (62) opposing the first free end (61) along the longitudinal axis (L) of the stamper (60), wherein the stamper (60) comprises a circular plate (63) at the second free end (62) extending perpendicular to the longitudinal axis (L) of the stamper (60).

7. Surgical tool according to one of the preceding claims, **characterized in that** the second element (20) comprises a second free end (22) opposing the first free end (21) of the second element (20) along the longitudinal axis (L) of the second element (20), wherein particularly said second free end (22) delimits an opening (221) through which said cannula (50) and/or said guiding element (30) can be inserted into the hollow space (201) of the second element (20), wherein particularly the second element comprises a portion (220) that broadens towards said second free end (22), particularly such that said portion (220) comprises at least in sections the shape of a truncated cone, and wherein particularly the second free end (22) of the second element (20) forms a circulating protrusion protruding from an outside of said portion (220).

8. Surgical tool according to one of the preceding claims, **characterized in that** the first element (10) comprises a second free end (12) opposing the first free end (11) of the first element (10) along its longitudinal axis (L), wherein particularly said second free end (12) delimits an opening (121), through which the second element (20) can be inserted into the hollow space (101) of the first element (10), wherein particularly the hollow space (101) of the first element (10) comprises a section (120), along which the hollow space (101) of the first element (10) broadens towards said opening (121) of the first element (10), particularly such that said section (120) of the hollow space (101) of the first element (10) comprises the shape of a truncated cone, particularly such that said section (120) of the hollow space (101) of the first element (10) is able to receive said portion (220) of the second element (20).

9. Surgical tool according to claim 8, **characterized in that** the second free end (12) of the first element (10) forms a circulating protrusion protruding from an outside of the shell (100) of the first element (10).

10. Surgical tool according to one of the preceding claims, **characterized in that** the shell (400) of the drill (40) comprises an outside, wherein a plurality of recesses (402) are formed in said outside of the shell (400) of the drill (40) along the longitudinal axis (L) of the drill (40), wherein particularly the recesses (402) are equidistantly arranged.

11. Surgical tool according to one of the preceding claims, **characterized in that** the drill (40) comprises a second free end (42) opposing the drill head (41) along the longitudinal axis (L) of the drill (40), wherein the drill (40) comprises a hexagonal cross sectional contour at the second free end (42), particularly for clamping of the drill (40) in a boring socket of a drilling machine.

12. Surgical tool according to one of the preceding claims, **characterized in that** the tool further comprises a closure element (700) being designed to be introduced into said borehole, particularly so as to retain bone cement that was injected into the borehole, wherein particularly the closure element (700) is designed to be introduced into said borehole in a folded state, wherein the closure element (700) is further designed to unfold at least partially when positioned in said borehole, particularly such that the closure element (700) presses against a surrounding wall of said borehole.

13. Surgical tool according to claims 1, 4 and 12, **characterized in that** the closure element (700) is designed to be pushed into the borehole through the cannula (50) with help of the stamper (60).

14. Surgical tool according to one of the claims 12 to 13, **characterized in that** the closure element (700) comprises an outer diameter (702) that corresponds to the outer diameter of the cannula (50).

15. Surgical tool according to claim 12 or one of the claims 13 to 14 when referred back to claim 12, **characterized in that** the closure element (700) comprises an outer contour (701) which comprises a serrated shape in said folded state and an octagonal shape in a completely unfolded state.

## Patentansprüche

1. Chirurgisches Instrument zur Durchführung eines chirurgischen Eingriffs an einem Knochen, insbesondere zur minimalinvasiven Verstärkung des Knochens, das Folgendes aufweist:
- ein erstes und ein zweites Element (10, 20), die sich jeweils entlang einer Längsachse (L) erstrecken, wobei die beiden Elemente (10, 20) jeweils einen Mantel (100, 200) aufweisen, der einen Hohlraum (101, 201) des jeweiligen Elements (10, 20) definiert, wobei das zweite Element (20) ausgestaltet ist, entlang der Längsachse (L) des ersten Elements in den Hohlraum (101) des ersten Elements (10) eingeführt zu werden, um die beiden Elemente (10, 20) in einem eingebetteten Zustand anzuordnen, in dem die beiden Elemente (10, 20) koaxial zueinander angeordnet sind und in dem der Mantel (100) des ersten Elements (10) den Mantel (200) des zweiten Elements (20) umschließt, wobei insbesondere das zweite Element (20), das in dem eingebetteten Zustand angeordnet ist, ausgestaltet ist, mit einem ersten freien Ende (21) des zweiten Elements (20) voran in Richtung des Knochens geschoben zu werden, um in Kontakt mit dem Knochen zu treten,
- ein Führungselement (30), das sich entlang einer Längsachse (L) erstreckt, wobei das Führungselement (30) ein erstes freies Ende (31) aufweist, und wobei das Führungselement (30) ausgestaltet ist, entlang der Längsachse (L) des zweiten Elements mit dem freien Ende (31) des Führungselements (30) voran in den Hohlraum (201) des zweiten Elements (20) eingeführt zu werden, um das Führungselement (30) in einem eingeführten Zustand in Bezug zum zweiten Element (20) anzuordnen, wenn dieses in dem eingebetteten Zustand angeordnet ist, wobei das Führungselement (30) in dem eingeführten Zustand koaxial zu dem zweiten Element (20) angeordnet ist und von dem Mantel (200) des zweiten Elements (20) umschlossen ist, und wobei das Führungselement (30) ausgestaltet ist, mit seinem ersten freien Ende (31) voran in den Knochen gebohrt zu werden, wenn es in dem eingeführten Zustand angeordnet ist, um das Führungselement (31) im Knochen zu verankern, und
- einen Bohrer (40), der sich entlang einer Längsache (L) erstreckt, wobei der Bohrer (40) einen Mantel (400) aufweist, der einen Hohlraum (401) des Bohrers (40) definiert, wobei der Bohrer (40), insbesondere nach Entfernen des zweiten Elements (20) aus dem ersten Element (10) und von dem Führungselement (30), dazu ausgestaltet ist, entlang der Längsachse (L) des Bohrers mit einem Bohrkopf (41) des Bohrers (40) voran auf das Führungselement (30) geschoben zu werden, um den Bohrer (40) in Bezug zum Führungselement (30) und zum ersten Element (10) in einem Bohrzustand anzuordnen, wobei in dem Bohrzustand das erste Element (10) den Bohrer (40) zum Schutz des Gewebes umschließt und das Führungselement (30) in dem Hohlraum (401) des Bohrers (40) angeordnet ist, um den Bohrer (40) zu führen, und wobei der Bohrkopf (41) ausgestaltet ist, im Bohrzustand ein Bohrloch in die Kortikalis und die Spongiosa des Knochens zu bohren,
**dadurch gekennzeichnet, dass**
das Instrument überdies eine Kanüle (50) aufweist, die sich entlang einer Längsachse (L) erstreckt, wobei die Kanüle (50) einen Mantel (500) aufweist, der einen Hohlraum (501) der Kanüle (50) definiert, durch den Knochenzement geleitet werden kann, der in das Bohrloch injiziert werden soll, wobei die Kanüle (50) ausgestaltet ist, entlang der Längsachse (L) des zweiten Elements (20) mit einem ersten freien Ende (51) der Kanüle (50) voran in den Hohlraum (201) des zweiten Elements (20) eingeführt zu werden, um die Kanüle (50) in einem Injektionszustand in Bezug zum zweiten Element (20) anzuordnen, insbesondere nach Entfernen des Führungselements (30) aus dem zweiten Element (20), wobei die Kanüle (50) im Injektionszustand koaxial zum zweiten Element (20) angeordnet ist, und von dem Mantel (200) des zweiten Elements (20) umschlossen ist.

2. Chirurgisches Instrument nach Anspruch 1, **dadurch gekennzeichnet, dass** das zweite Element (20), insbesondere nach Entfernen des Bohrers (40) und des ersten Elements (10) von dem Führungselement (30) und Neuanordnung des zweiten Elements (20) über dem Führungselement (30), so dass dieses wieder im Hohlraum (201) des zweiten Elements (20) angeordnet ist, ausgestaltet ist, mit seinem ersten freien Ende (21) voran entlang der Längsachse (L) des zweiten Elements in das Bohrloch eingeführt zu werden, insbesondere um die Kortikalis des Knochens zu überbrücken.

3. Chirurgisches Instrument nach Anspruch 1 oder 2, **dadurch gekennzeichnet, dass** die Kanüle (50) so ausgestaltet ist, dass sie mit ihrem ersten freien Ende (51) entlang der Längsachse (L) des zweiten Elements aus dem Hohlraum (201) des zweiten Elements (20) hervorsteht, insbesondere in die Spongiosa des Knochens, wobei insbesondere das freie erste Ende (51) der Kanüle (50) eine Injektionsöffnung (151) der Kanüle (50) begrenzt, durch die Knochenzement aus dem Hohlraum (501) der Kanüle (50) abgegeben werden kann.

4. Chirurgisches Instrument nach Anspruch 1 oder 3, **dadurch gekennzeichnet, dass** das Instrument überdies einen Pressstempel (60) aufweist, der sich entlang einer Längsachse (L) erstreckt, wobei der Pressstempel (60) ausgestaltet ist, entlang der Längsachse (L) der Kanüle mit einem ersten freien Ende (61) des Pressstempels (60) voran in den Hohlraum (501) der Kanüle (50) eingeführt zu werden, um Knochenzement, der sich in dem Hohlraum (501) der Kanüle (50) befindet, aus dem Hohlraum (501) zu schieben, insbesondere durch das Bohrloch hindurch in die Spongiosa des Knochens.

5. Chirurgisches Instrument nach Anspruch 4, **dadurch gekennzeichnet, dass** der Pressstempel (60) und die Kanüle (50) ausgestaltet sind, entlang der Längsachse (L) des zweiten Elements im Hohlraum (201) des zweiten Elements (20) zusammen aus einer zurückgezogenen ersten Position zu einer vorgeschobenen zweiten Position bewegt zu werden, insbesondere, um Knochenzement, der in das Bohrloch injiziert wurde, in die Spongiosa zu schieben, wodurch in der Spongiosa des Knochens ein hohles kreiszylindrisches Knochenzementimplantat gebildet wird, wobei insbesondere das zweite Element (20) ausgestaltet ist, die Kanüle (50) und den Pressstempel (60) bei der Bewegung aus der zurückgezogenen Position in die vorgeschobene Position zu führen.

6. Chirurgisches Instrument nach Anspruch 4 oder 5, **dadurch gekennzeichnet, dass** der Pressstempel (60) ein zweites freies Ende (62) aufweist, das dem ersten freien Ende (61) entlang der Längsachse (L) des Pressstempels (60) gegenüberliegt, wobei der Pressstempel (60) am zweiten freien Ende (62) eine kreisförmige Platte (63) aufweist, die sich senkrecht zur Längsachse (L) des Pressstempels (60) erstreckt.

7. Chirurgisches Instrument nach einem der vorhergehenden Ansprüche, **dadurch gekennzeichnet, dass** das zweite Element (20) ein zweites freies Ende (22) aufweist, das dem ersten freien Ende (21) des zweiten Elements (20) entlang der Längsachse (L) des zweiten Elements (20) gegenüberliegt, wobei insbesondere das zweite freie Ende (22) eine Öffnung (221) begrenzt, durch die die Kanüle (50) und/oder das Führungselement (30) in den Hohlraum (201) des zweiten Elements (20) eingeführt werden können, wobei insbesondere das zweite Element ein Teilstück (220) aufweist, das sich in Richtung des zweiten freien Endes (22) verbreitert, insbesondere derart, dass das Teilstück (220) zumindest abschnittsweise die Form eines Kegelstumpfs aufweist, und wobei insbesondere das zweite freie Ende (22) des zweiten Elements (20) einen umlaufenden Vorsprung bildet, der von einer Außenseite des Teilstücks (220) hervorsteht.

8. Chirurgisches Instrument nach einem der vorhergehenden Ansprüche, **dadurch gekennzeichnet, dass** das erste Element (10) ein zweites freies Ende (12) aufweist, das dem ersten freien Ende (11) des ersten Elements entlang seiner Längsachse (L) gegenüberliegt, wobei insbesondere das zweite freie Ende (12) eine Öffnung (121) begrenzt, durch die das zweite Element (20) in den Hohlraum (101) des ersten Elements (10) eingeführt werden kann, wobei insbesondere der Hohlraum (101) des ersten Elements (10) einen Abschnitt (120) aufweist, entlang dessen der Hohlraum (101) des ersten Elements (10) sich in Richtung der Öffnung (121) des ersten Elements (10) verbreitert, insbesondere derart, dass der Abschnitt (120) des Hohlraums (101) des ersten Elements (10) die Form eines Kegelstumpfs aufweist, insbesondere derart, dass der Abschnitt (120) des Hohlraums (101) des ersten Elements (10) das Teilstück (220) des zweiten Elements (20) aufnehmen kann.

9. Chirurgisches Instrument nach Anspruch 8, **dadurch gekennzeichnet, dass** das zweite freie Ende (12) des ersten Elements (10) einen umlaufenden Vorsprung bildet, der auf einer Außenseite des Mantels (100) des ersten Elements (10) hervorsteht.

10. Chirurgisches Instrument nach einem der vorhergehenden Ansprüche, **dadurch gekennzeichnet, dass** der Mantel (400) des Bohrers (40) eine Außenseite aufweist, wobei entlang der Längsachse (L) des Bohrers (40) eine Vielzahl von Ausnehmungen (402) in der Außenseite des Mantels (400) des Bohrers (40) ausgebildet ist, wobei insbesondere die Ausnehmungen (402) in gleichen Abständen angeordnet sind.

11. Chirurgisches Instrument nach einem der vorhergehenden Ansprüche, **dadurch gekennzeichnet, dass** der Bohrer (40) ein zweites freies Ende (42) aufweist, das dem Bohrkopf (41) entlang der Längsachse (L) des Bohrers (40) gegenüberliegt, wobei der Bohrer (40) am zweiten freien Ende (42) eine hexagonale Querschnittskontur aufweist, insbesondere zum Einklemmen des Bohrers in ein Bohrfutter einer Bohrmaschine.

12. Chirurgisches Instrument nach einem der vorhergehenden Ansprüche, **dadurch gekennzeichnet, dass** das Instrument überdies ein Verschlusselement (700) aufweist, das ausgestaltet ist, in das Bohrloch eingebracht zu werden, insbesondere um Knochenzement, der in das Bohrloch injiziert wurde, zurückzuhalten, wobei insbesondere das Verschlusselement (700) ausgestaltet ist, in einem gefalteten Zustand in das Bohrloch eingebracht zu werden, wobei das Verschlusselement (700) überdies ausgestaltet ist, sich zumindest teilweise zu entfalten, wenn es in dem Bohrloch positioniert ist, insbesondere derart, dass das Verschlusselement (700) gegen eine umgebende Wand des Bohrlochs drückt.

13. Chirurgisches Instrument nach Anspruch 1, 4 und 12, **dadurch gekennzeichnet, dass** das Verschlusselement (700) ausgestaltet ist, mithilfe des Pressstempels (60) durch die Kanüle (50) in das Bohrloch geschoben zu werden.

14. Chirurgisches Instrument nach einem der Ansprüche 12 bis 13, **dadurch gekennzeichnet, dass** das Verschlusselement (700) einen Außendurchmesser (702) aufweist, der dem Außendurchmesser der Kanüle (50) entspricht.

15. Chirurgisches Instrument nach Anspruch 12 oder einem der Ansprüche 13 bis 14, wenn auf Anspruch 12 rückbezogen, **dadurch gekennzeichnet, dass** das Verschlusselement (700) eine Außenkontur (701) aufweist, die im gefalteten Zustand eine eingekerbte Form und im vollständig entfalteten Zustand eine achteckige Form aufweist.

## Revendications

1. Outil chirurgical pour effectuer une chirurgie osseuse, plus particulièrement pour renforcer l'os avec une effraction minimale, comprenant :
- un premier et un second élément (10, 20) s'étendant le long d'un axe longitudinal (L), respectivement, dans lequel les deux éléments (10, 20) comprennent chacun une enveloppe (100, 200) qui délimite une cavité (101, 201) de l'élément respectif (10, 20), dans lequel le second élément (20) est conçu pour être inséré dans la cavité (101) du premier élément (10) le long de l'axe longitudinal (L) du premier élément pour placer les deux éléments (10, 20) dans un état imbriqué, dans lequel les deux éléments (10, 20) sont placés coaxialement l'un par rapport à l'autre et dans lequel l'enveloppe (100) du premier élément (10) couvre l'enveloppe (200) du second élément (20), dans lequel en particulier le second élément (20) étant placé dans ledit état imbriqué est conçu pour être poussé avec une première extrémité libre (21) du second élément (20) devant vers l'os de manière à entrer en contact avec ledit os,
- un élément de guidage (30) s'étendant le long d'un axe longitudinal (L), dans lequel l'élément de guidage (30) comprend une première extrémité libre (31), et dans lequel l'élément de guidage (30) est conçu pour être inséré dans la cavité (201) du second élément (20) le long de l'axe longitudinal (L) du second élément avec ladite première extrémité libre (31) de l'élément de guidage (30) devant, pour placer l'élément de guidage (30) dans un état inséré par rapport au second élément (20) quand ce dernier est placé dans ledit état imbriqué, dans lequel état inséré l'élément de guidage (30) est placé coaxialement par rapport au second élément (20) et recouvert par l'enveloppe (200) du second élément (20), et dans lequel l'élément de guidage (30) est conçu pour être introduit par forage dans ledit os avec sa première extrémité libre (31) devant lorsque placé dans ledit état inséré de manière à ancrer ledit élément de guidage (31) dans ledit os, et
- un foret (40) s'étendant le long d'un axe longitudinal (L), lequel foret (40) comprend une enveloppe (400) qui définit une cavité (401) du foret (40), dans lequel le foret (40), en particulier après avoir retiré le second élément (20) du premier élément (10) et de l'élément de guidage (30), est conçu pour être placé sur l'élément de guidage (30) le long de l'axe longitudinal (L) du foret avec une tête de foret (41) du foret (40) devant pour placer le foret (40) dans un état de forage par rapport à l'élément de guidage (30) et au premier élément (10), dans lequel état de forage le premier élément (10) recouvre ledit foret (40) pour la protection du tissu et l'élément de guidage (30) est placé dans la cavité (401) du foret (40) de manière à guider ledit foret (40), et dans lequel la tête de foret (41) est conçue pour forer un trou de forage dans la partie corticale et la partie spongieuse dudit os dans ledit état de forage,
**caractérisé en ce que**
l'outil comprend en outre une canule (50), laquelle canule (50) s'étend le long d'un axe longitudinal (L), dans lequel la canule (50) comprend une enveloppe (500) qui définit une cavité (501) de la canule (50), à travers laquelle un ciment osseux peut être envoyé pour injection dans ledit trou de forage, dans lequel la canule (50) est conçue pour être insérée dans la cavité (201) du second élément (20) le long de l'axe longitudinal (L) du second élément avec une première extrémité libre (51) de la canule (50) devant pour placer la canule (50) dans un état d'injection par rapport au second élément (20), en particulier après que l'élément de guidage (30) a été retiré du second élément (20), dans lequel état d'injection la canule (50) est placée coaxialement par rapport au second élément (20) et recouverte par l'enveloppe (200) du second élément (20).

2. Outil chirurgical selon la revendication 1, **caractérisé en ce que** le second élément (20), en particulier après le retrait du foret (40) et du premier élément (10) de l'élément de guidage (30) et le replacement du second élément (20) sur l'élément de guidage (30) de sorte que ce dernier est placé de nouveau dans la cavité (201) du second élément (20), est conçu pour être inséré avec sa première extrémité libre (21) devant dans ledit trou de forage de forage le long de l'axe longitudinal (L) du second élément, en particulier de manière à relier la partie corticale dudit os.

3. Outil chirurgical selon la revendication 1 ou 2, **caractérisé en ce que** la canule (50) est conçue pour faire saillie avec sa première extrémité libre (51) hors de la cavité (201) du second élément (20) le long de l'axe longitudinal (L) du second élément, en particulier dans la partie spongieuse de l'os, dans lequel en particulier ladite première extrémité libre (51) de la canule (50) délimite une ouverture d'injection (151) de la canule (50), à travers laquelle le ciment osseux peut être évacué hors de la cavité (501) de la canule (50).

4. Outil chirurgical selon la revendication 1 ou 3, **caractérisé en ce que** l'outil comprend en outre une matrice de pressage (60) qui s'étend le long d'un axe longitudinal (L), dans lequel la matrice de pressage (60) est conçue pour être insérée dans la cavité (501) de la canule (50) le long de l'axe longitudinal (L) de la canule avec une première extrémité libre (61) de la matrice de pressage (60) devant, pour pousser le ciment osseux qui réside dans la cavité (501) de la canule (50) hors de ladite cavité (501), en particulier par ledit trou de forage dans la partie spongieuse de l'os.

5. Outil chirurgical selon la revendication 4, **caractérisé en ce que** la matrice de pressage (60) et la canule (50) sont conçues pour être déplacées ensemble dans la cavité (201) du second élément (20) le long de l'axe longitudinal (L) du second élément d'une première position rétractée dans une seconde position avancée, en particulier de manière à pousser le ciment osseux injecté dans ledit trou de forage dans la partie spongieuse, formant ainsi un implant cylindrique circulaire creux de ciment osseux dans la partie spongieuse dudit os, dans lequel en particulier ledit second élément (20) est conçu pour guider la canule (50) et la matrice de pressage (60) lors dudit mouvement de la position rétractée dans la position avancée.

6. Outil chirurgical selon la revendication 4 ou 5, **caractérisé en ce que** la matrice de pressage (60) comprend une seconde extrémité libre (62) opposée à la première extrémité libre (61) le long de l'axe longitudinal (L) de la matrice de pressage (60), dans lequel la matrice de pressage (60) comprend une plaque circulaire (63) à la seconde extrémité libre (62) qui s'étend perpendiculairement à l'axe longitudinal (L) de la matrice de pressage (60).

7. Outil chirurgical selon l'une quelconque des revendications précédentes, **caractérisé en ce que** le second élément (20) comprend une seconde extrémité libre (22) opposée à la première extrémité libre (21) du second élément (20) le long de l'axe longitudinal (L) du second élément (20), dans lequel en particulier ladite seconde extrémité libre (22) délimite une ouverture (221) à travers laquelle ladite canule (50) et/ou ledit élément de guidage (30) peuvent être insérés dans la cavité (201) du second élément (20), dans lequel en particulier le second élément comprend une partie (220) qui s'élargit vers ladite seconde extrémité libre (22), en particulier de manière à ce que ladite partie (220) comprend au moins en sections la forme d'un cône tronqué, et dans lequel en particulier la seconde extrémité libre (22) du second élément (20) forme une saillie de circulation faisant saillie à partir de l'extérieur de ladite partie (220).

8. Outil chirurgical selon l'une quelconque des revendications précédentes, **caractérisé en ce que** le premier élément (10) comprend une seconde extrémité libre (12) opposée à la première extrémité libre (11) du premier élément (10) le long de son axe longitudinal (L), dans lequel en particulier ladite seconde extrémité libre (12) délimite une ouverture (121), à travers laquelle le second élément (20) peut être inséré dans la cavité (101) du premier élément (10), dans lequel en particulier la cavité (101) du premier élément (10) comprend une section (120) le long de laquelle la cavité (101) du premier élément (10) s'élargit vers ladite ouverture (121) du premier élément (10), en particulier de sorte que ladite section (120) de la cavité (101) du premier élément (10) adopte la forme d'un cône tronqué, en particulier de sorte que ladite section (120) de la cavité (101) du premier élément (10) peut recevoir ladite partie (220) du second élément (20).

9. Outil chirurgical selon la revendication 8, **caractérisé en ce que** la seconde extrémité libre (12) du premier élément (10) forme une saillie de circulation faisant saillie à partir de l'extérieur de l'enveloppe (100) du premier élément (10).

10. Outil chirurgical selon l'une quelconque des revendications précédentes, **caractérisé en ce que** l'enveloppe (400) du foret (40) comprend un extérieur, dans lequel une pluralité de creux (402) sont formés dans ledit extérieur de l'enveloppe (400) du foret (40) le long de l'axe longitudinal (L) du foret (40), dans lequel en particulier les creux (402) sont placés de manière équidistante.

11. Outil chirurgical selon l'une quelconque des revendications précédentes, **caractérisé en ce que** le foret (40) comprend une seconde extrémité libre (42) opposée à la tête de foret (41) le long de l'axe longitudinal (L) du foret (40), dans lequel le foret (40) comprend un contour en section transversale hexagonale à la seconde extrémité libre (42), en particulier pour le serrage du foret (40) dans la douille de fraisage d'une perceuse.

12. Outil chirurgical selon l'une quelconque des revendications précédentes, **caractérisé en ce que** l'outil comprend en outre un élément de fermeture (700) conçu pour être introduit dans ledit trou de forage, en particulier de manière à retenir le ciment osseux qui a été injecté dans le trou de forage, dans lequel en particulier l'élément de fermeture (700) est conçu pour être introduit dans ledit trou de forage dans un état plié, dans lequel l'élément de fermeture (700) est en outre conçu pour se déplier au moins partiellement lorsque positionné dans ledit trou de forage, en particulier de sorte que l'élément de fermeture (700) presse contre une paroi environnante dudit trou de forage.

13. Outil chirurgical selon les revendications 1, 4 et 12, **caractérisé en ce que** l'élément de fermeture (700) est conçu pour être poussé dans le trou de forage à travers la canule (50) avec l'aide de la matrice de pressage (60).

14. Outil chirurgical selon l'une des revendications 12 et 13, **caractérisé en ce que** l'élément de fermeture (700) possède un diamètre extérieur (702) qui correspond au diamètre extérieur de la canule (50).

15. Outil chirurgical selon la revendication 12 ou l'une des revendications 13 et 14 quand elles font référence à la revendication 12, **caractérisé en ce que** l'élément de fermeture (700) possède un contour extérieur (701) qui comporte une forme dentelée dans ledit état plié et une forme octogonale dans un état complètement déplié.
